# EUROPEAN PATENT APPLICATION

(11) **EP 2 392 325 A1**
(43) Date of publication of application: **07.12.2011**
(21) Application number: 10005816.3
(22) Date of filing: 04.06.2010
(51) Int. Cl.: A61K 31/353, A61P 19/02

(54) **Compounds for the prevention and/or treatment of osteoarthrosis**

(71) Applicant: Universitätsklinikum Münster, 48149 Münster (DE)
(72) Inventor: The designation of the inventor has not yet been filed
(74) Representative: Schiweck, Weinzierl & Koch

(57) **Abstract**

The present invention relates to new compounds which are inter alia derivable from hops for use in the treatment of (for treating)/prevention or healing of a disease which is associated with an excess transport of hyaluronan across a lipid bilayer, in particular a disease which is associated with or characterized by degeneration and/or a destruction of cartilage (and/or for the prevention of aggrecan loss). Food products comprising these compounds for use in the treatment (for treating) a disease which is associated with an excess transport of hyaluronan across a lipid bilayer, in particular a disease which is associated with or characterized by degeneration and/or a destruction of cartilage, are also envisaged.

## Description

The present invention relates to new compounds which are inter alia derivable from hops for use in the treatment of (for treating)/prevention or healing of a disease which is associated with an excess transport of hyaluronan across a lipid bilayer, in particular a disease which is associated with or characterized by degeneration and/or a destruction of cartilage (and/or for the prevention of aggrecan loss). Food products comprising these compounds for use in the treatment (for treating) a disease which is associated with an excess transport of hyaluronan across a lipid bilayer, in particular a disease which is associated with or characterized by degeneration and/or a destruction of cartilage, are also envisaged.

### Specification

Hyaluronan is the major water binding component of the extracellular matrix. It is a very large glycosaminoglycan that is exported into the extracellular matrix by fibroblasts or epithelial cells, where it attracts water up to 99 % of its own weight, swells to enormous volumes and displaces other resident macromolecules [1]. The growing hyaluronan chain is synthesised within the cytoplasm and exported into the extracellular matrix where water attraction and swelling occurs. We investigated hyaluronan exporters in human fibroblasts and identified MRP5 [8; 9].

In recent years it has become evident that cellular hyaluronan synthesis plays an important role in shedding and displacement of other components such as removal of antibodies or phagocytes from virulent Streptococci [10], detachment of fibroblasts during mitosis [11], tumour metastasis [12], as well as proteoglycan loss from osteoarthritic joints [13; 14]. Due to the enormous hydration volume, hyaluronan will replace any other components from its site of origin, when it extrudes from plasma membranes [1]. In humans, synthesis and degradation of hyaluronan is a very dynamic process. A total amount of hyaluronan of 34 mg is turned over in the circulation of an adult human daily [30; 31]. Aberrant hyaluronan synthesis will lead to disturbances of cell behaviour and tissue integrity and hydration. The following pathological disturbances are accompanied by a hyaluronan overproduction.

### Metastasis and Cancer

Most malignant solid tumours contain elevated levels of hyaluronan. Enrichment of hyaluronan in tumours may be caused by increased production by tumours themselves or by induction in the surrounding stroma cells [19; 58]. Also many human tumors are characterized by an overproduction of hyaluronan such as melanoma [89a], mesothelioma [117a] or colon carcinoma [118a]. Because hyaluronan production is correlated with cell proliferation [86a], inhibition of hyaluronan transport will also reduce tumour growth.

### Inflammation and Edema

Inflammation of various organs is often accompanied with an accumulation of hyaluronan. This can cause edema due to the osmotic activity and can lead to dysfunction of the organs. For example, hyaluronan accumulation in the rheumatoid joint impedes the flexibility of the joint [62]. Hyaluronan accumulation in rejected transplanted kidneys can cause edema and increased intracapsular pressure [63]. It also accumulates in pulmonary edema [64] and during myocardial infarction [65].

### Other diseases

Most injuries are followed by inflammation and hyaluronan overproduction that may lead to severe health problems. Excess hyaluronan production is observed after organ transplantation that may lead to tissue rejection, and after a heart infarct. It is also associated with alveolitis, pancreatitis, pulmonary or hepatic fibrosis, radiation induced inflammation, Crohn's disease, myocarditis, scleroderma, psoriasis, sarcoidosis [119a-135a]. Overproduction of hyaluronan is also the cause of lump formation after contusion or insect bites, and therefore it will be possible to inhibit swelling by the inhibitors of hyaluronan transport. Large amounts of hyaluronan accumulate in demyelinated lesions in areas of multiple sclerosis [66] or ischemic stroke [67].

### Osteoarthritis

Osteoarthrosis which is also known as degenerative arthritis or degenerative joint disease, is characterized by cartilage erosion, proteolysis of aggrecan and collagen and disturbed synthesis rates of aggrecan and hyaluronan by chondrocytes, hyaluronan overproduction being an early reaction. The destruction of joint cartilage is the major cause of human arthritic diseases, i.e., osteoarthrosis. Chondrocytes represent only 5% of the tissue and are responsible for cartilage synthesis. It consists of two main components: the network of type II collagen, which provides the tensile strength and stiffness, and the large aggregating proteoglycan, aggrecan, which is responsible for the osmotic swelling capability and elasticity. Aggrecan decorates a backbone of hyaluronan that is anchored in the plasma membrane of chondrocytes at the hyaluronan synthase and further bound by the cell surface receptor CD44. The biosynthesis of hyaluronan and proteoglycans has different mechanisms and occur in different compartments [5]. Proteoglycans are synthesized in the Golgi and exocytosed by vesicles. Hyaluronan is polymerized at the inner side of plasma membranes [3-5] and exported by ABC-transporters [8]. Both components aggregate in the extracellular matrix [68] with up to 200 aggrecan molecules decorating one hyaluronan chain [69]. In healthy cartilage, the hyaluronan and aggrecan are synthesized and degraded at similar rates [70], whereas the turnover of collagens is much slower [71]. The proteoglycan residue is liberated from the hyaluronan binding region by aggrecanase [72]. Most of hyaluronan is removed by endocytosis through the CD44 receptor in healthy cartilage [73] or liberated into the environment in osteoarthritic cartilage [74]. Aggrecan leaves cartilage either as intact molecule or after proteolysis depending on the stimulus [75]. Aggregate formation is important from the physiological point of view, since it ensures the retention of aggrecan within the collagen network.

Key events in osteoarthritic cartilage (and therefore also in osteoarthritis) are increased hyaluronan, decreased aggrecan synthesis [74; 76] and proteolytic cleavage of collagen type II and aggrecan core protein [77; 78]. For a long time, it was thought that proteolytic degradation of collagen and aggrecan was the primary event in cartilage breakdown. Many efforts to develop protease inhibitors led to compounds that were chondroprotective in vitro or in animal models [79-82], but results from clinical trials were equivocal [83; 84]. Recently, we discovered that a variety of multidrug resistance inhibitors interferes with hyaluronan export from human cells, and their inhibition profile suggested that the multidrug resistance associated protein MRP5 could be a hyaluronan exporter [8]. The drugs prevented aggrecan loss from chondrocyte cell cultures, cartilage organ cultures and in an animal model of osteoarthrosis indicating that hyaluronan overproduction was involved in aggrecan loss [13]. We extended the analysis to the effects of the hyaluronan export inhibitors zaprinast, vardenafil and tadalafil to collagen degradation of II-1a activated bovine cartilage explants. The drugs normalized proteoglycan content and collagen degradation and reduced infiltration of degrading enzymes. Thus inhibition of hyaluronan export is an appropriate target for therapeutic intervention in osteoarthrosis [14] (see also WO2005/013947).

### Arthritis

Arthritis (from Greek arthro-, joint and -itis, inflammation; plural: arthritides) is a group of conditions involving damage to the joints of the body. There are over 100 different forms of arthritis. Arthritis forms are rheumatoid arthritis, psoriatic arthritis, and autoimmune diseases in which the body attacks itself. Septic arthritis is caused by joint infection. The major complaint by individuals who have arthritis is pain. Pain is often a constant and daily feature of the disease. The pain may be localized to the back, neck, hip, knee or feet. The pain from arthritis occurs due to inflammation that occurs around the joint, damage to the joint from disease, daily wear and tear of joint, muscles strains caused by forceful movements against stiff, painful joints and fatigue. Until now academic and industrial research spends enormous efforts for the development of protease inhibitors for treatment of arthritis. However, protease inhibitors do not inhibit the primary process. Arthritis as such is thus difficult to treat. Up to now no therapy exists that can alter the course of the disease or can repair existing damages. Treatment is confined to pain relieve by physiotherapy, analgetic or anti-inflammatory drugs or intraarticularly applied hyaluronan. Taken together, no disease modifying arthritic drugs are available.

The technical problem underlying the present invention is to provide means and methods for treating, healing and/or preventing diseases which are associated with an excess transport of hyaluronan across a lipid bilayer, in particular diseases which are associated with or characterized by degeneration and/or a destruction of cartilage.

The solution to this technical problem is achieved by providing the embodiments characterized in the claims.

A variety of documents is cited throughout this specification. The disclosure content of said documents (including any manufacturer's specifications, instructions etc.) is herewith incorporated by reference; however, there is no admission that any document cited is indeed prior art as to the present invention.

It must be noted that as used herein, the singular forms "a", "an", and "the", include plural references unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

In a first aspect, the present invention relates to compounds of the formulas (I): 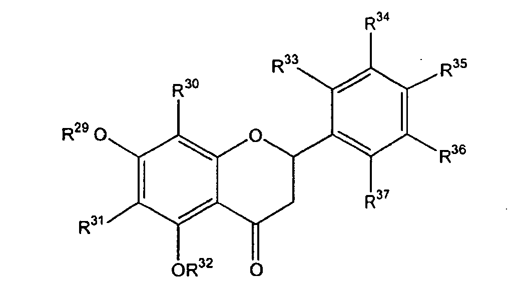 wherein
R29 is H or CH₃
R30 is H or an alkyl-group, such as CH₃, CH₂-CH=CH(CH₃)₂
R31 is H
R32 is H or CH₃O
R33 is H, OH or CH₃O
R34 is H, OH or CH₃O
R35 is H, OH or CH₃O
R36 is H, OH or CH₃O
R37 is H, OH or CH₃O
or a pharmaceutically acceptable salt thereof,
and/or (II) 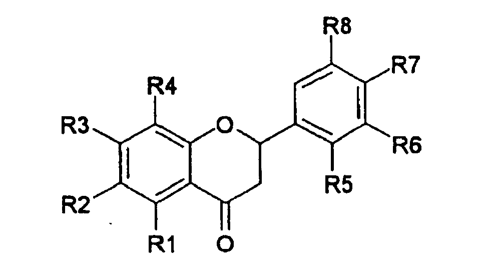 wherein
R1 is H or CH₃O
R2 is H
R3 is H or CH₃
R4 is H or an alkyl-group, such as CH₃, CH₂-CH=CH(CH₃)₂
R5 is H, OH or CH₃O
R6 is H, OH or CH₃O
R7 is H, OH or CH₃O
R8 is H, OH or CH₃O
or a pharmaceutically acceptable salt thereof.
and/or (III) 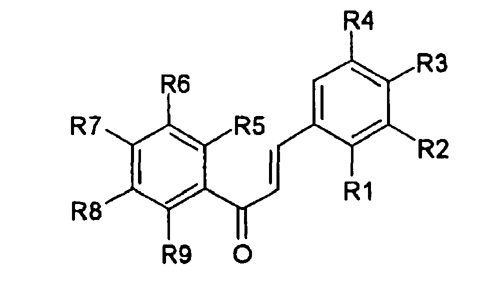 wherein
R1 is H, OH or CH₃O
R2 is H, OH or CH₃O
R3 is H, OH or CH₃O
R4 is H, OH or CH₃O
R5 is H, OH or CH₃O
R6 is H or an alkyl-group, such as CH₃, CH₂-CH=CH(CH₃)₂
R7 is H or CH₃
R8 is H, OH or CH₃O
R9 is H, OH or CH₃O
or a pharmaceutically acceptable salt thereof,
for use in the treatment, prevention (prophylaxis) or healing of a disease which is
(a) associated with or characterized by an excess transport of hyaluronan across a lipid bilayer; and/or
(b) associated with or characterized by degeneration and/or a destruction of cartilage, preferably in the joint (more preferably the synovial joint); and/or
(c) associated with or characterized by proteoglycan (preferably aggrecan) loss, preferably in the joint (more preferably the synovial joint); and/or
(d) associated with or characterized by proteolytic cleavage of collagen type II and aggrecan core protein, preferably in the joint (more preferably the synovial joint).

In a preferred embodiment, said disease is osteoarthritis. Said disease was already discussed in great detail herein before.

"Healing" means not necessarily a complete reversion of the disease but includes that the cause for the disease (which is (a) an excess transport of hyaluronan across a lipid bilayer; and/or (b) degeneration and/or a destruction of cartilage, preferably in the joint (more preferably the synovial joint); and/or (c) aggrecan loss, preferably in the joint (more preferably the synovial joint); and/or (d) proteolytic cleavage of collagen type II and aggrecan core protein, preferably in the joint (more preferably the synovial joint)) is at least reverted to some extent. This includes for example the repair of existing damages (for example the desrtruction of cartilage as specificed above), a decreased hyaluoronan transport, a decreased aggrecan loss, a decreased proteolytic cleavage of collagen type II and aggrecan core protein. It is preferred that the mentioned "repair" takes place in the joint (more preferably the synovial joint).

The term "alkyl" as used herein, whether used alone or as part of another group, refer to substituted or unsubstituted aliphatic hydrocarbon chains, the difference being that alkyl groups are monovalent (i. e., terminal) in nature. Alkyl include, but is not limited to, straight and branched chains containing from 1 to about 12 carbon atoms, preferably 1 to 6 carbon atoms, unless explicitly specified otherwise. For example, methyl, ethyl, propyl, isopropyl, butyl, i-butyl and t- butyl are encompassed by the term "alkyl." Specifically included within the definition of "alkyl" are those aliphatic hydrocarbon chains that are optionally substituted. Representative optional substituents include, but are not limited to, hydroxy, oxo (=0) and prenyl. The carbon number as used in the definitions herein refers to carbon backbone and carbon branching, but does not include carbon atoms of the substituents, such as alkoxy substitutions and the like.

"Pharmaceutically acceptable salts" includes both organic and inorganic salts (e.g. with alkali and alkaline earth metals, ammonium, ethanolamine, diethanolamine and meglumine, chloride, hydrogen carbonate, phosphate, sulphate and acetate counterions). Appropriate pharmaceutically acceptable salts are well described in the pharmaceutical literature. In addition, some of these salts may form solvates with water or organic solvents such as ethanol. Such solvates are also included within the scope of this invention.

In a preferred embodiment, the above mentioned compounds are Isoxanthohumol (A), 8-Prenylnaringenin (B) and/or Xanthohumol (C)

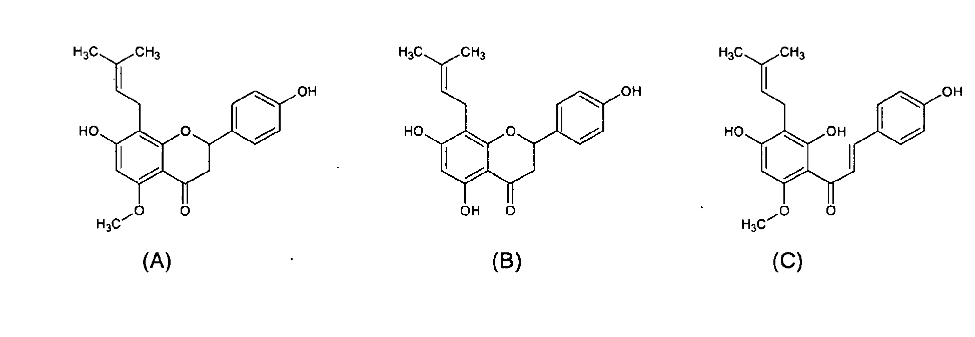

It could be demonstrated that the compounds of the present invention are able to revert, treat or prevent the above mentioned causes of the disease and are thus sutiable for the medical purposes described herein (see the appended examples which are incorporated herewith into the specification).

The compounds of the invention may be used alone or in any conceivable combination. The same equates to the compounds which are defined herein in more general terms.

The herein described inhibitors have been isolated from hops (hops extract). This isolated extract contains at least 3% by weight of prenylflavonoids, preferably Isoxanthohumol, 8-Prenylnaringenin and Xanthohumol. It is however also possible to synthesize these compounds by means and methods which are well known to the skilled person. Methods for preparing e.g. xanthohumol-rich hop composition, methods for synthesizing e.g. xanthohumol and methods for enriching hop extracts in prenylated flavonoids are well-known and are described in e.g. WO 2009/023710, WO 2009/026206, WO 2005/058336, DE 19939350, WO 03/014287, WO 02/085393, WO 02/085393, EP 0679393, EP 1543834 and US 2005/0019438, all of which are incorporated herein.

Hop (Humulus lupulis L.) is a climbing perennial vine that vigorously grows 20-35 feet each year. Humulus lupus is a member of the hemp family, which has grown wild since ancient times in Europe, Asia, and North America. The female flowers mature in late summer have been used for centuries as a bittering agent in the brewing of beer. Hops contain alpha acids such as humulone, co-humuone, ad-humulone, and beta acids such as lupulone and co-lupulone. In the last few years, the plant has gained increasing attention as a source of prenylflavonoids, a flavonoid subclass containing an apolar prenyl-side chain attached to one of the phenolic rings. These are present in the lupulin glands, found at the base of the bracteoles in the hop cones of the female plant. Of these prenylflavonoids two chalcones (xanthohumol and desmethylxanthohumol) and three flavanones (isoxanthohumol, 8-prenylnaringenin and 6-prenylnaringenin), now receive much attention because of their potential health-promoting properties. In ancient times the young shoots of hops were eaten as a vegetable and the dried flowers were used for their slight narcotic effect and sedative action in the treatment of mania, toothache, earache, and neuralgia [177]. Modern herbal medicine practitioners continue to use hops as a sedative and mild hypnotic, as well as for its endocrine, free radical scavenging, and antitumor properties.

The majority of hops' medicinal actions have been attributed to its flavonoid constituents. Flavonoids are abundant throughout nature and exert a broad range of biological activities in plants and animals. There are now considered to be over 4,000 flavonoids existent in nature. Some of the biological activities of flavonoids include anti-inflammatory, antiviral, antifungal, antibacterial, estrogenic, anti-oxidant, antiallargenic, anticarcinogenic, and antiproliferative medicinal properties.

Flavonoids are composed of different chemical classes such as flavones, isoflavones, flavonols, flavanols, flavanones, and chalcones. These compounds differ in the level of oxidation of the flavane nucleus and in the number and position of hydroxyl, methyl, and methoxyl substituents. [175] Six chalcones (xanthohumol, 2',4',6',4-tetrahydroxy-3'-prenylchalcone, 2',4',6',4-tetrahydroxy-3'-geranylchalcone, 5'-prenylxanthohumol, dehydrocycloxanthohumol, and dehydrocycloxanthohumol hydrate) and three flavanones (isoxanthohumol, 6-prenylnaringenin and 8-prenylnaringenin) have been isolated from the hops plant. [176]

The prenylflavonoids from hops and in particular xanthohumol have been intensively studied and were found to have beneficial effects on some diseases including chronic allergic contact dermatitis [157], hepatoma carcinoma proliferation [158], hepatic inflammation and fibrosis [159], and chronic lymhocytic leukemia [160]. They are anti-inflammatory [161], antimutagenic [162], antiinvasive [163] and antigenotoxic [164]. Xanthohumol has been shown to modulate other cellular targets such as TNF-α release [165], suppression of NFkappaB regulated gene products [166], inhibition of topoisomerase [167] and alkaline phosphatase [168].

Xanthohumol is the principal prenylated chalcone present in the female inflorescences of the hop plant Humulus lupulus L. (Cannabaceae). Xanthohumol readily undergoes thermally-driven cyclization to the flavone isoxanthohumol during the brewing process [169, 170]. Xanthohumol is a yellow-orange substance with a melting point of 172 degrees C. A typical ethanol extract of hops yields about 3 mg./g (3%) of xanthohumol out of a total flavonoid content of 3.46 mg/g. Dried hop contains about 0.2 to 1.0 % by weight xanthohumol. Increasing the levels of xanthohumol may lead to hops varieties that have enhanced health-promoting properties. Xanthohumol is present as a predominant prenylchalcone in the female hop cones in concentrations up to 1% (w/w) [173]. Knowledge of the genes encoding the enzymes of xanthohumol biosynthesis may allow production of xanthohumol in alternative host organisms, such as bacteria. Xanthohumol has been shown to be a very potent cancer chemopreventive compound in vitro with an exceptional broad spectrum of inhibitory mechanisms at the initiation, promotion, and progression stages of carcinogenesis [171].

Isoxanthohumol is a natural product produced from hops. Isoxanthohumol is the hop polyphenol produced from xanthohumol. Isoxanthohumol can be used as an ingredient in food, cosmetics or pharmaceutical applications. Isoxanthohumol is moderately estrogenic in vitro. Xanthohumol can be converted to Isoxanthohumol through acid-catalyzed cyclization in the stomach. [174]

8- Prenylnaringenin is the most potent phytoestrogen thus far identified. It therefore has potential as a selective estrogen receptor modulator (SERM) for treatment of osteoporosis and other menopausal/post-menopausal conditions.

The inhibitory profile for hyaluronan export of three particularily preferred compounds of the present invention is depicted in the table below (IC50 for hyaluronan export).

| Compound | Structure | Inhibition of hyaluronan export IC₅₀ (µM) |
|---|---|---|
| Isoxanthohumol | 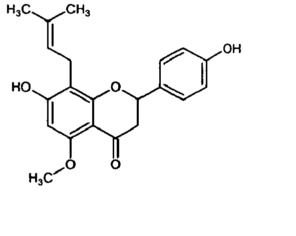 | 15 |
| Xanthohumol | 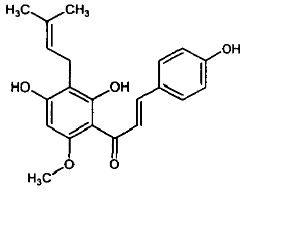 | 8 |
| 8-Prenylnaringenin | 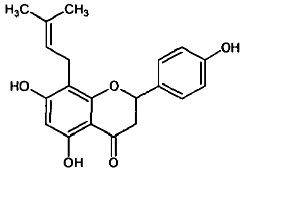 | 15 |

The present invention also relates to an inhibitor based on Isoxanthohumol, 8-Prenylnaringenin and/or Xanthohumol "Based on" means a chemically altered derivative of one of these three compounds, which derivative has a comparable biological function when compared with Isoxanthohumol, 8-Prenylnaringenin or Xanthohumol. "Comparable biological function" means that the chemical derivatives of the invention are able to reduce the hyaluronan export with a deviation of the reducing activity in respect to one of the compounds on which they are based on (Isoxanthohumol, 8-Prenylnaringenin or Xanthohumol) of not more than about 40%, 30%, 20%, 15%, 10%, 5%, 2,5%, 2% or 1% (10% or less being preferred), for example under conditions which equate to or are in essence identical with those set out in Example 1. "Comparable biological function" does alternatively mean that the IC50 of hyaluronan transport activity of the chemically altered derivatives of the invention deviates not more than about 40%, 30%, 20%, 15%, 10%, 5%, 2,5%, 2% or 1% (10% pr less being preferred) from the IC50 of one of the inhibitors selected from Isoxanthohumol, 8-Prenylnaringenin or Xanthohumol. WO2005/013947 discloses further suitable assays to evaluate the hyaluronan export.

It is assumed that the inhibitors of the present invention bind, preferably specifically, to the MRP5-transporter (see appended example 3). MRP5 is an ABC-transporter which is described in great detail for example in WO2005/013947 and elsewehre. It is thus envisaged that the compounds of the invention reduce the MRP5-mediated hyaluronan transport rate about 5%, 10%, 20%, 30%, 40%, 50%, 60%, 70%, 80%, 90% or even 100% when compared to the transport rate that is achieved without the addition of said compound. One specific screening assay for the hyaluronan transporter is based on the extrusion of labelled hyaluronan oligosaccharides from intact cells in monolayer culture. Said assay is further explained in WO2005/013947, particularly in the appended examples of said document (e. g Example 8 or Example 11) as well as in appended example 3 of the present invention. In such cases it is sufficient to analyse the effect of the inhibitor e.g. on a cell comprising MRP5, i.e. one compares the hyaluronan-transport before and after the addition of the inhibitor and thereby identifies inhibitors which reduce the transport-rate of hyaluronan across a lipid bilayer.

It is preferred that the compounds of the invention specifically reduce(s) the transport of hyaluronan across a lipid bilayer mediated by MRP5. The term "specifically reduce(s)" used in accordance with the present invention means that the inhibitor specifically causes a reduction of the transport of hyaluronan as mediated by MRP5 but has no or essentially has no significant effect on other cellular proteins (e.g. other ABC-transporters) or enzymes. The inhibitors can be discriminated by virtue of their binding to MRP5. Methods have been described that assay the binding of inhibitors to ABC transporters [92a; 93a]. One specific screening assay for the hyaluronan transport as mediated by the ABC-transporter MRP5 is based on the extrusion of labeled hyaluronan oligosaccharides from intact cells in monolayer culture (see e.g. Example 11 of WO2005/013947). Alternatively, liposomes can be employed which encompass MRP5 in the lipid bilayer.

The pharmaceutical composition of the present invention may optionally comprise a pharmaceutical carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. The dosage regimen will be determined by the attending physician and clinical factors.

Upon using the compounds (or compositions) of the present invention, it is possible to treat, ameliorate, heal and/or prevent diseases, which are
(a) associated with or characterized by an excess transport of hyaluronan across a lipid bilayer; and/or
(b) associated with or characterized by degeneration and/or a destruction of cartilage, preferably in the joint (more preferably the synovial joint); and/or
(c) associated with or characterized by proteoglycan (preferably aggrecan) loss, preferably in the joint (more preferably the synovial joint); and/or
(d) associated with or characterized by proteolytic cleavage of collagen type II and aggrecan core protein, preferably in the joint (more preferably the synovial joint).

The skilled person is well aware which specific diseases are characterized for example by an excess level of hyaluronan at the exterior of cells and, provided with the teaching and disclosure of the present invention can easily test for such an excess hyaluronan transport. Thus, it is for example possible to identify a subject at risk for a disease which is associated with an excess transport of hyaluronan across a lipid bilayer or to diagnose a disease which is associated with an excess transport of hyaluronan across a lipid bilayer. This can be diagnosed e.g., by evaluating cells from an individual which cells are provided in a form which facilitates an invitro diagnostic analysis. Such cells can be collected from body fluids, skin, hair, biopsies and other sources. Chondrocytes represent only 5% of the tissue but they are responsible for synthesizing and controlling the matrix (including the hyaluronan production). It is therefore preferred that the mentioned cells include chondrocytes. The remaining parameters mentioned herein (characterizing the diseases to be treated etc.) may be evaluated by a physician or a skilled person for example in biopsy material or alternative by means and methods which allow the evaluation of whether (b) the cartilage is degenerated and/or destroyed (preferably in the joint, more preferably in the synovial joint); and/or (c) there is proteoglycan (preferably aggrecan) loss (preferably in the joint, more preferably the synovial joint); and/or (d) whether there is proteolytic cleavage of collagen type II and aggrecan core protein, preferably in the joint (more preferably the synovial joint). An compound of the present invention is preferably a compound which is characterized by a selection of one or more of the following capabilities: reduction of the overall transport of hyaluronan across a lipid-bilayer; reduction and/or reversion of the destruction of cartilage; maintenance of the actual state of destruction of cartilage; prevention of a further destruction of the cartilage etc.

The term "excess transport" as used herein means that the transport of hyaluronan exceeds the transport level as compared with a normal/natural state of a comparable control-cell/subject. It has to be understood that in the context of the present invention, the terms "transport" and export" are used interchangeably. The term "normal/natural state of a comparable control-cell/subject" means the transport-rate of hyaluronan in a control-cell which is preferably of the same nature as the test-cell (e.g. both cell are chondrocytes) but which is derived from a different source. "A different source" includes e.g. a cell/tissue sample obtained from a healthy subject who does not suffer from a disease defined herein or a cell/tissue sample obtained from a distinct joint of the same subject wherein said different joint appears to be free from associated symptoms of a disease defined herein.

It is also envisaged that the compounds of the present invention (as defined herein before) are used for the preparation of a pharmaceutical composition for the treatment, prevention (prophylaxis) or healing of a disease which is
(a) associated with or characterized by an excess transport of hyaluronan across a lipid bilayer; and/or
(b) associated with or characterized by degeneration and/or a destruction of cartilage, preferably in the joint (more preferably the synovial joint); and/or
(c) associated with or characterized by proteoglycan (preferably aggrecan) loss, preferably in the joint (more preferably the synovial joint); and/or
(d) associated with or characterized by proteolytic cleavage of collagen type II and aggrecan core protein, preferably in the joint (more preferably the synovial joint).

The term "degeneration and/or destruction of cartilage" includes within the meaning of the present invention dysregulation of turnover and repair of joint tissue. The pathological features are focal areas of destruction of articular cartilage associated with hypertrophy of the subcondral bone, joint margin and capsule. The radiological changes include joint space narrowing, subchondral sclerosis and cysts, pain, loss of joint motion and disability. Diseases/conditions which may be treated with the compounds of the invention can be exemplified as follows: ischemic or inflammatory edema; tumors which are characterized by an overproduction of hyaluronan such as melanoma [89a], mesothelioma [117a] or colon carcinoma [118a]; lump formation after contusion or insect bites; injuries/conditions which are followed by inflammation and hyaluronan overproduction like heart infarct, alveolitis, pancreatitis, pulmonary or hepatic fibrosis, radiation induced inflammation, Crohn's disease, myocarditis, scleroderma, psoriasis, sarcoidosis [119a-135a]. Ischemic edema is caused by increased hyaluronan production around blood vessels that leads to vessel constriction and reduced oxygen supply. This is thought to be the main cause of death after a heart attack. Therefore immediate application of drugs inhibiting hyaluronan production will improve these conditions. Tissue necrosis will lead to inflammation with increased hyaluronan production that in turn causes edema. To break this vicious cycle inhibitors of hyaluronan transport will expand the choice for medical treatments.

It is however particularily preferred that the compounds of the present invention are used for the treatment, prevention (prophylaxis) or healing of arthritis. "Arthritis" includes, but is not limited to, osteoarthritis, (juvenile) chronic arthritis, rheumatoid arthritis, psoriatic arthritis, A. *mutilans,* septic arthritis, infectious arthritis and/or reactive arthritis. Thus, the term "arthritis" as used herein includes all forms of arthritis, e. g. the primary or idiopathic form.

In a most preferred embodiment of the present invention the compounds of the present invention are used for the treatment, prevention (prophylaxis) or healing of osteoarthritis (also known as osteoarthrosis or "non-inflammatory arthritis"). Said disease is a type of arthritis that is caused by the breakdown and eventual loss of the cartilage of one or more joints.

The inhibitors of the present invention can be applied prophylactically, for example with subjects that have or might have an enhanced individual risk factor such as obesity, heredity, for women after the menopause, osteoporosis, hypermobility, for persons with distorted joint shape or for persons with repetitive use of particular joint groups.

It is also envisaged that the compounds of the invention are used in a therapeutically effective amount/concentration, i.e. in an amount/concentration that is sufficient to exert its effect.

It is also envisaged that the compounds of the present invention are employed in co-therapy approaches, i.e. in co-administration with other medicaments or drugs, for example other drugs for preventing, treating or ameliorating osteoarthritis.

The present invention also relates to a method of preventing, ameliorating and/or treating the symptoms of a disease which is
(a) associated with or characterized by an excess transport of hyaluronan across a lipid bilayer; and/or
(b) associated with or characterized by degeneration and/or a destruction of cartilage, preferably in the joint (more preferably the synovial joint); and/or
(c) associated with or characterized by proteoglycan (preferably aggrecan) loss, preferably in the joint (more preferably the synovial joint); and/or
(d) associated with or characterized by proteolytic cleavage of collagen type II and aggrecan core protein, preferably in the joint (more preferably the synovial joint).

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease. The present invention is directed towards treating patients with medical conditions relating to a disease which is associated with an excess transport of hyaluronan across a lipid bilayer, e.g. arthritis. Accordingly, a treatment of the invention would involve preventing, inhibiting or relieving any medical condition related to a disease which is associated with an excess transport of hyaluronan across a lipid bilayer, e.g. arthritis. As mentioned elsewhere before, said arthritis is e.g. characterized by degeneration and/or a destruction of cartilage. Osteoarthritis is particularily preferred.

In the context of the present invention the term "subject" means an individual in need of a treatment of an affective disorder. Preferably, the subject is a mammalian, particularly preferred a human, a horse, a camel, a dog, a cat, a pig, a cow, a goat or a fowl. A human is most preferred.

The term "administered" means administration of a therapeutically effective dose of the inhibitors and/or test-compounds as disclosed herein. By "therapeutically effective amount" is meant a dose that produces the effects for which it is administered. The exact dose will depend on the purpose of the treatment, and will be ascertainable by one skilled in the art using known techniques. The administration of the pharmaceutical composition can be done in a variety of ways as discussed above, including, but not limited to, orally, subcutaneously, intravenously, intra-arterial, intranodal, intramedullary, intrathecal, intraventricular, intranasally, intrabronchial, transdermally, intranodally, intrarectally, intraperitoneally, intramuscularly, intrapulmonary, vaginally, rectally, or intraocularly. In some instances, for example, in the treatment of wounds and inflammation, the candidate agents may be directly applied as a solution dry spray.

Oral administration is particularily preferred. The compounds (composition comprising the compounds) may be in solid, liquid or gaseous form and may be, inter alia, in the form of (a) powder(s), (a) tablet(s), (a) film preparation(s), (a) solution(s) (an) aerosol(s), granules, pills, suspensions, emulsions, capsules, syrups, liquids, elixirs, extracts, tincture or fluid extracts or in a form which is particularly suitable for oral administration. Liquid preparations suitable for oral administration, for example syrups can be prepared, using water, conventional saccharides such as sucrose, sorbitol and fructose, glycols such as polyethylene glycol and propylene glycol, oils such as sesame seed oil, olive oil and soybean oil, antiseptics such as p-hydroxybenzoate ester, preservatives such as p-hydroxybenzoate derivatives, for example p-hydroxybenzoate methyl and sodium benzoate, and other materials such as flavors, for example strawberry flavor or peppermint. Further, preparations suitable for oral administration, for example tablets, powders and granules can be produced, using conventional saccharides such as sucrose, glucose, mannitol, and sorbitol, starch such as potato, wheat and corn, inorganic materials such as calcium carbonate, calcium sulfate, sodium hydrogen carbonate, and sodium chloride, plant powders such as crystal cellulose, licorice powder and gentian powder, excipients such as pinedex, disintegrators such as starch, agar, gelatin powder, crystal cellulose, carmellose sodium, carmellose calcium, calcium carbonate, sodium hydrogen carbonate and sodium alginate, lubricants such as magnesium stearate, talc, hydrogenated vegetable oils, macrogol, and silicone oil, binders such as polyvinyl alcohol, hydroxypropyl cellulose, methyl cellulose, ethyl cellulose, carmellose, gelatin, and starch glue fluid, surfactants such as fatty acid ester, and plasticizers such as glycerin. A film preparation(s) can be prepared by methods known in the art.

In accordance with the present invention it is also envisaged to employ the compounds of the invention comprised in foodstuff. The term "foodstuff' encompasses all eatable and drinkable food and drinks. These are, for example, gum, spray, beverage, candies, infant formula, ice cream, frozen dessert, sweet salad dressing, milk preparations, cheese, quark, lactose-free yogurt, acidified milk, coffee cream or whipped cream and the like. Specific foods or drinks, to which the active ingredient is added, include, for example, juices, refreshing drinks, soups, teas, beer (non-alcoholic, reduced alcoholic and alcoholic); sour milk beverages, dairy products such as fermented milks, ices, butter, cheese, processed milk and skim milk, meat products such as ham, sausage, and hamburger, fish meat cake products, egg products such as seasoned egg rolls and egg curd, confectioneries such as cookie, jelly, snacks, and chewing gum, breads, noodles, pickles, smoked products, dried fishes and seasonings. The form of the food or drink includes, for example, powder foods, sheet-like foods, bottled foods, canned foods, retort foods, capsule foods, tablet foods and fluid foods.

Foodstuff comprising some of the compounds of the present invention is also described in great detail in WO03090555, which is incorporated herein by way of reference.

Most preferred is the oral administration in form of beverages. Even more preferred is beer (non-alcoholic, reduced alcoholic and alcoholic). Such beverages including a process for their production have been disclosed in great detail in EP-B1 1 431 385 which is incorporated herein by reference. Xanthohumol containing beer is meanwhile well-known under the brandname Xan™. Such beverages may contain up to 10mg Xanthohumol/liter which might already be sufficient to exert the effect described in the context of the present invention.

The compositions, compounds, uses and methods of the present invention are applicable to both human therapy and veterinary applications.

This disclosure may best be understood in conjunction with the accompanying drawings, incorporated herein by references. Furthermore, a better understanding of the present invention and of its many advantages will be had from the following examples, given by way of illustration and are not intended as limiting.

### The figures show:

**Fig. 1** Inhibition of hyaluronan export from bovine chondrocytes and explants of bovine cartilage. The concentrations were related to 100% of the control. The error bars indicate the sd of four determinations.
**Fig**. **2** Fluorescein export by MRP5. In the absence (■) or presence of xanthohumol (◊), isoxanthohumol (□) or 8-prenylnaringenin (□). The sd of three determinations were below 2 %.
**Fig. 3** Inhibition of proteoglycan loss. In the guanidinium hydrochloride solubilized explants (A) and in the culture media (B). The concentrations were related to 100% of the control. The error bars indicate the sd of four determinations.
**Fig. 4** Inhibition of collagen degradation
**Fig. 5** Inhibition of gelatinase liberation

### Examples:

The following examples illustrate the invention. These examples should not be construed as to limit the scope of this invention. The examples are included for purposes of illustration and the present invention is limited only by the claims.

### Example 1: Assay for hyaluronan transport/export inhibitors in fibroblast cell culture

Trypsinised fibroblasts were suspended in Dulbeccós medium at 10⁵ cells/ml and 100 µl aliquots were transferred to a 96 well microtiter plate. The first row received 200 µl of the suspension and 20 µl of the inhibitors of the invention dissolved in phosphate buffered saline at concentrations of 4 mM. A serial dilution of the inhibitors was established by transfer of 100 µl aliquots from the first row to the following rows. All experiments were performed in duplicates. The last row did not receive any inhibitor and served as control. The cells were incubated for 2 days at 37°C and aliquots (5 and 20 µl) of the culture medium were used for measurement of the hyaluronan concentration in the cell culture medium by an ELISA [125]. Briefly, the wells of a 96 well Covalink-NH-microtiter plate (NUNC) were coated with 100 µl of a mixture of 100 mg/ml of hyaluronan (Healon^{®}), 9,2 µg/ml of N-Hydroxysuccinimide-3-sulfonic acid and 615 µl/ml of 1-ethyl-3-(3-dimethylaminopropyl)-carbodiimide for 2 hours at room temperature and overnight at 4°C. The wells were washed three times with 2 M NaCl, 41 mM MgSO₄, 0.05% Tween-20 in 50 mM phosphate buffered saline pH 7.2 (buffer A) and once with 2 M NaCl, 41 mM MgS0₄, in phosphate buffered saline pH 7.2. Additional binding sites were blocked by incubation with 300 µl of 0.5 % bovine serum albumin in phosphate buffered saline for 30 min at 37°C. Calibration of the assay was performed with standard concentrations of hyaluronan ranging from 15 ng/ml to 6000 ng/ml in equal volumes of culture medium as used for measurement of the cellular supernatants. A solution (50 µl) of the biotinylated hyaluronan binding fragment of aggrecan (Calbiochem) in 1.5 M NaCl, 0.3 M guanidinium hydrochloride, 0,08% bovine serum albumin 0.02% NaN₃ 25 mM phosphate buffer pH 7.0 was preincubated with 50 µl of the standard hyaluronan solutions or cellular supernatants for 1 hour at 37°C. The mixtures were transferred to the hyaluronan-coated test plate and incubated for 1 hour at 37°C. The microtiter plate was washed three times with buffer A and incubated with 100 µl /well of a solution of streptavidin-horseraddish-peroxidase (Amersham) at a dilution of 1:100 in phosphate buffered saline, 0.1 % Tween-20 for 30 min at room temperature. The plate was washed five times with buffer A and the colour was developed by incubation with a 100 µl/well of a solution of 5 mg o-phenylenediamine and 5 µl 30% H₂O₂ in 10 ml of 0.1 M citrate-phosphate buffer pH 5.3 for 25 min at room temperature. The adsorption was read at 490 nm. The concentrations in the samples were calculated from a logarithmic regression curve of the hyaluronan standard solutions.

### Example 2:

### Inhibition of hyaluronan export from bovine chondrocytes and explants of bovine cartilage

Bovine chondrocytes in alginate beads (A) were incubated in the absence and presence of II-17 and increasing concentrations of xanthohumol (open bars), isoxanthohumol (left hatched bars), and 8-prenylnaringenin (vertical hatched bars) for 6 days and the hyaluronan concentrations were determined in the supernatant. The concentrations were related to 100% of the control. Bovine cartilage explants (B) were similarly incubated, weighed and hyaluronan concentrations were determined. The concentrations were related to 100% of the control. The error bars indicate the sd of four determinations.

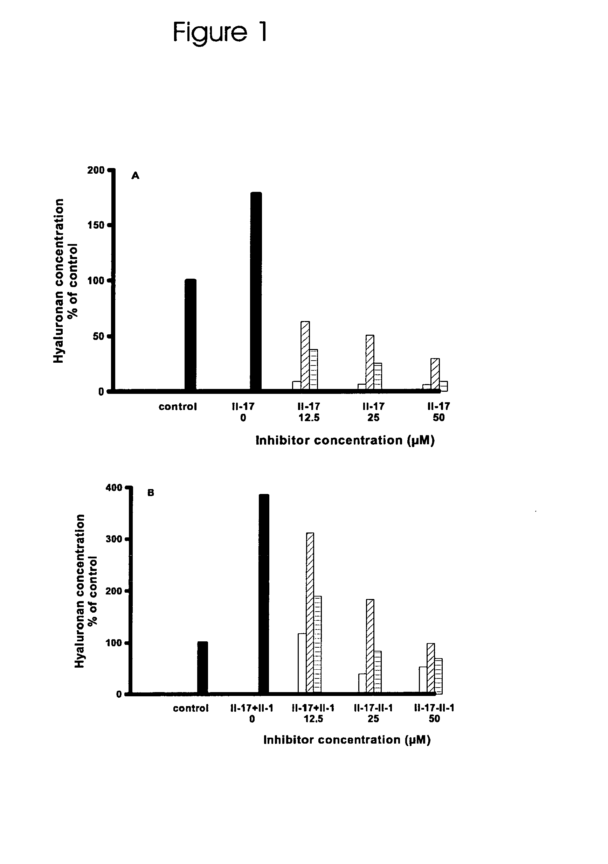

### See Fig. 1

### Example 3:

### Fluorescein export by MRP5

Inhibition of the MRP5 Substrate Fluorescein Export by Cytosolic Hyaluronan Oligosaccharides:
Normal HEK293 or MRP5-overexpressing HEK293 cells were trypsinized and incubated in RPMI medium containing 0.5 M sucrose, 10% polyethylene glycol 600, and hyaluronan or
hyaluronan oligosaccharides at concentrations of 100 µg/ml for 15 min at 37 °C. Cells were then placed in a mixture of RPMI/H2O 1:2 (v/v) for 5 min to lyse the pinocytic vesicles. The loading and lysis procedure was repeated once. Afterward the cells (2 x 10⁶ cells/ml) were incubated for 10 min on ice with an equal volume of fluorescein diacetate (4 µM in phosphate-buffered saline). Cells were centrifuged and resuspended in ice-cold phosphate-buffered saline containing 5.6 mM glucose, 1 mM MgCl2, and 5 mM KCI. The suspension was incubated at 37 °C; aliquots were taken at different time points, centrifuged, and the fluorescence of the supernatant was determined at 485 and 528 nm by a Synergy HT reader (Biotek Instruments). MRP5 overexpressing HEK cells were incubated with non-fluorescent fluorescein diacetate which is intracellularly cleaved to the MRP5 substrate fluorescein in the absence (■) or presence of xanthohumol (◊), isoxanthohumol (□) or 8-prenylnaringenin (□). The sd of three determinations were below 2 %.

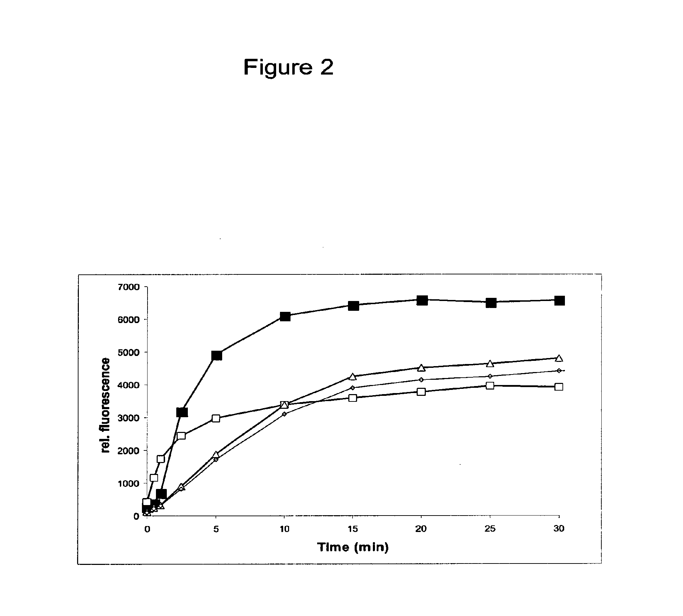

### See Fig. 2

### Example 4:

### Inhibition of proteoglycan loss

Cartilage explants were weighed (average wet weight 20 mg) and incubated in media (control) incubated or in media containing an osteoarthritis inducing mixture of II-17 in the presence of increasing concentrations of the prenylflavonoids for 5 days. The tissues were extracted with 1.5 ml of a solution of 4 mol/I guanidinium hydrochloride, 0.1 mol/l ε-aminohexanoid acid, 5 mmol/I benzamidine, 10 mmol/I N-ethylmaleinimide and 0.5 mmol/l phenalmethylsulfonyl fluoride for 3 days at 4°C. The solution was centrifuged for 5 minutes at 10.000 g and the proteoglycans were determined in the supernatant using the alcian blue method, as described previously (Bjornsson S: Simultaneous preparation and quantitation of proteoglycans by precipitation with alcian blue. Anal Biochem 1993, 210:282-291).

The proteoglycan concentrations were determined after 3 days in the guanidinium hydrochloride solubilized explants (A) and in the culture media (B). The concentrations were related to 100% of the control. The error bars indicate the sd of four determinations.

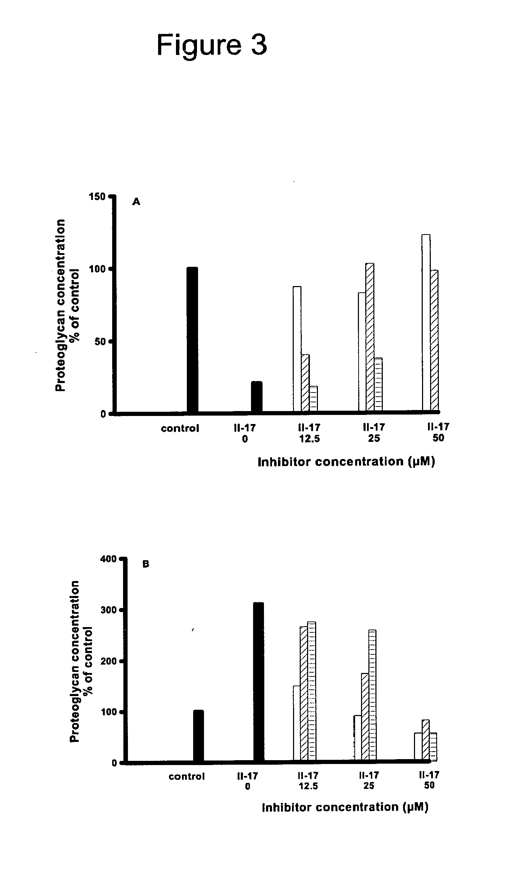

### See Fig. 3

### Example 5:

### Inhibition of collagen degradation

Cartilage explants were incubated with and without a mixture of II-1α, II-1β and II-17 for 14 days Collagen was visualized by the van Giesson stain.

Cartilage explants were incubated with and without a mixture of 10 ng/ml of II-1α, 10 ng/ml of ll-1β and 25 ng/ml of II-17 for 14 days in the presence of 12.5 µM or 50 µM 8-prenylnaringenin, xanthohumol, or isoxanthohumol. The addition of the cytokines was repeated daily. The tissues were fixed with 3.7 % paraformaldehyde for 24 hours, imbedded in paraffin and stained by the van Giesson method. (1. Bring sections to distilled water, 2. Stain nuclei with Celestin Blue 5 mins, 3. Rinse in distilled water, 4. Stain in haematoxylin 5 mins, 5. Wash well in running tap water 5 mins, 6. Flood with Curtis stain 5 mins, 7. Blot, 8. Dehydrate rapidly in alcohols, clear and mount.)

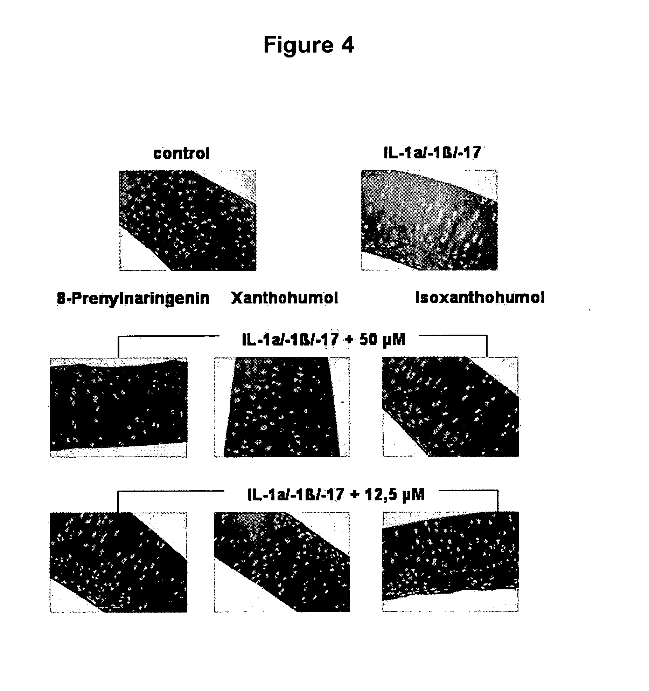

### See Fig. 4

### Example 6:

### Inhibition of gelatinase liberation

Chondrocytes in alginate beads were incubated in the absence or presence of 25 ng/ml II-17 and the prenylflavonoids xanthohumol, isoxanthohumol and 8-prenylnaringenin at concentrations of 12.5 µM and 50 µM for 3 days at 37°C. The activity of gelatin degrading enzymes released into the culture supernatant was determined by zymography.

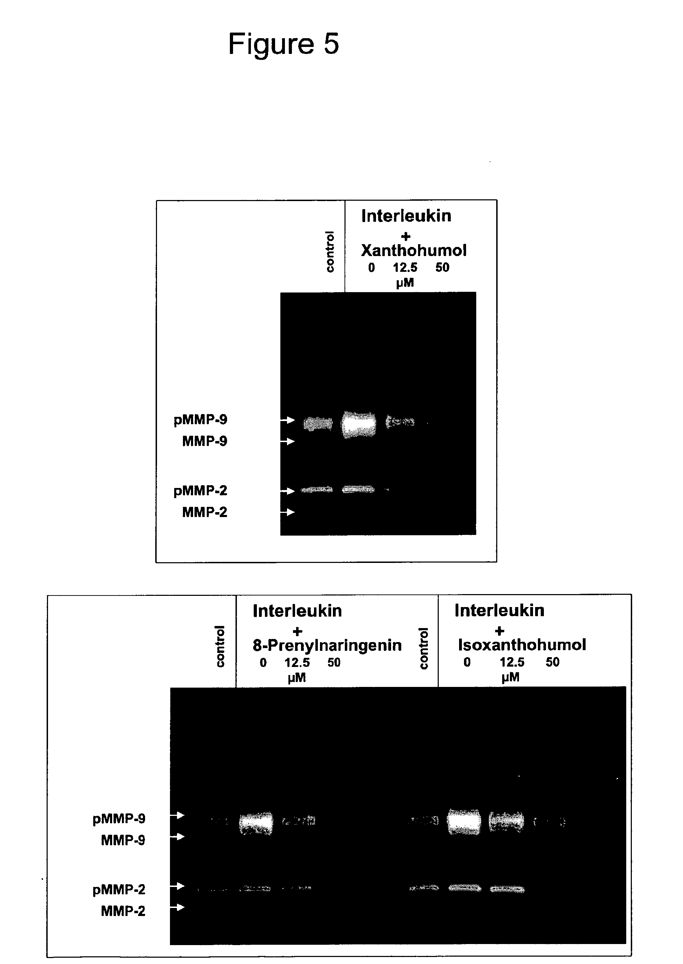

### See Fig. 5

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, detailed Description, and Examples is hereby incorporated herein by reference.

### References

[1] Laurent,T.C. (1964) The interaction between polysaccharides and other macromolecules. The exclusion of molecules from hyaluronic acid gels and solutions. Biochem. J, 93, 106-112.
[3] Prehm,P. (1983) Synthesis of hyaluronate in differentiated teratocarcinoma cells. Mechanism of chain growth. Biochem. J., 211, 191-198.
[4] Prehm,P. (1983) Synthesis of hyaluronate in differentiated teratocarcinoma cells. Characterization of the synthase. Biochem. J., 211, 181-189.
[5] Prehm,P. (1984) Hyaluronate is synthesized at plasma membranes. Biochem. J., 220, 597-600.
[8] Prehm,P. & Schumacher,U. (2004) Inhibition of hyaluronan export from human fibroblasts by inhibitors of multidrug resistance transporters. Biochem. Pharmacol., 68, 1401-1410.
[9] Schulz,T., Schumacher,U., & Prehm,P. (2007) Hyaluronan export by the ABC-transporter MRP5 and its modulation by intracellular cGMP. J Biol. Chem., 282, 20999-21004.
[10] Nickel,V., Prehm,S., Lansing,M., Mausolf,A., Podbielski,A., Deutscher,J., & Prehm,P. (1998) An ectoprotein kinase of group C streptococci binds hyaluronan and regulates capsule formation. J. Biol. Chem., 273, 23668-23673.
[11] Brecht,M., Mayer,U., Schlosser,E., & Prehm,P. (1986) Increased hyaluronate synthesis is required for fibroblast detachment and mitosis. Biochem. J., 239, 445-450.
[12] Lüke,H.J. & Prehm,P. (1999) Synthesis and shedding of hyaluronan from plasma membranes of human fibroblasts and metastatic and non-metastatic melanoma cells. Biochem. J., 343, 71-75.
[13] Prehm,P. (2005) Inhibitors of hyaluronan export prevent proteoglycan loss from osteoarthritic cartilage. J. Rheumatol., 32, 690-696.
[14] Deiters,B. & Prehm,P. (2008) Inhibition of hyaluronan export reduces collagen degradation in IL-1 treated cartilage. Arthritis Res. Ther., 10, R8.
[19] Toole,B.P., Biswas,C., & Gross,J. (1979) Hyaluronate and invasiveness of the rabbit V2 carcinoma. Proc. Natl. Acad. Sci. U.S.A., 76, 6299-6303.
[30] Fraser,J.R. & Laurent,T.C. (1989) Turnover and metabolism of hyaluronan. Ciba. Found. Symp., 143,41-53.
[31] Lebel,L., Gabrielsson,J., Laurent,T.C., & Gerdin,B. (1994) Kinetics of circulating hyaluronan in humans. Eur. J. Clin. Invest., 24, 621-626.
[58] Knudson,W., Biswas,C., & Toole,B.P. (1984) Interactions between human tumor cells and fibroblasts stimulate hyaluronate synthesis. Proc. Natl. Acad. Sci. U. S. A., 81, 6767-6771.
[62] Engström-Laurent,A. (1997) Hyaluronan in joint disease. J. Intern. Med., 242, 57-60.
[63] Hällgren,R., Gerdin.B., & Tufveson,G. (1990) Hyaluronic acid accumulation and redistribution in rejecting rat kidney graft. Relationship oto the transplantation edema. J. Exp. Med., 171, 2063-2076.
[64] Nettelbladt,O., Tengblad,A., & Hällgren,R (1989) Lung accumulation of hyaluronan parallels pulmonary edema in experimental alveolitis. Am. J. Physiol., 257, L379-L384.
[65] Waldenstrom,A., Martinussen,H.J., Gerdin,B., & Hällgren,R. (1991) Accumulation of hyaluronan and tissue edema in experimental myocardial infarction. J. Clin. Invest., 88, 1622-1628.
[66] Back,S.A., Tuohy,T.M., Chen,H., Wallingford,N., Craig,A., Struve,J., Luo,N.L., Banine,F., Liu,Y., Chang,A., Trapp,B.D., Bebo,B.F., Rao,M.S., & Sherman,L.S. (2005) Hyaluronan accumulates in demyelinated lesions and inhibits oligodendrocyte progenitor maturation. Nat. Med.*.*
[67] Al'qteishat,A., Gaffney,J., Krupinski,J., Rubio,F., West,D., Kumar,S., Kumar,P., Mitsios,N., & Slevin,M. (2006) Changes in hyaluronan production and metabolism following ischaemic stroke in man. Brain., 129, 2158-2176.
[69] Tang,L.H., Buckwalter,J.A., & Rosenberg,L.C. (1996) Effect of link protein concentration on articular cartilage proteoglycan aggregation. J. Orthop. Res., 14, 334-339.
[70] Morales,T.I. & Hascall,V.C. (1988) Correlated metabolism of proteoglycans and hyaluronic acid in bovine cartilage organ cultures. J. Biol. Chem., 263, 3632-3638.
[71] Maroudas,A., Palla,G., & Gilav,E. (1992) Racemization of aspartic acid in human articular cartilage. Connect. Tissue Res., 28, 161-169.
[72] Embry,J.J. & Knudson,W. (2003) G1 domain of aggrecan cointernalizes with hyaluronan via a CD44-mediated mechanism in bovine articular chondrocytes. Arthritis Rheum., 48, 3431-3441.
[73] Hua,Q., Knudson,C.B., & Knudson,W. (1993) Internalization of hyaluronan by chondrocytes occurs via receptor-mediated endocytosis. J. Cell Sci., 106, 365-375.
[74] D'Souza,A.L., Masuda,K., Otten,L.M., Nishida,Y., Knudson,W., & Thonar,E.J. (2000) Differential Effects of Interleukin-1 on Hyaluronan and Proteoglycan Metabolism in Two Compartments of the Matrix Formed by Articular Chondrocytes Maintained in Alginate. Arch. Biochem. Biophys., 374, 59-65.
[75] Sztrolovics,R., White,R.J., Roughley,P.J., & Mort,J.S. (2002) The mechanism of aggrecan release from cartilage differs with tissue origin and the agent used to stimulate catabolism. Biochem. J., 362, 465-472.
[76] Nishida,Y., D'Souza,A.L., Thonar,E.J., & Knudson,W. (2000) Stimulation of hyaluronan metabolism by interleukin-1alpha in human articular cartilage. Arthritis Rheum., 43, 1315-1326.
[77] Smith,R.L. (1999) Degradative enzymes in osteoarthritis. Front Biosci., 4, D704-D712.
[78] Poole,A.R., Nelson,F., Dahlberg,L., Tchetina,E., Kobayashi,M., Yasuda,T., Laverty,S., Squires,G., Kojima,T., Wu,W., & Billinghurst,R.C. (2003) Proteolysis of the collagen fibril in osteoarthritis. Biochem. Soc. Symp., 70, 115-123.
[79] Bottomley,K.M., Borkakoti,N., Bradshaw,D., Brown,P.A., Broadhurst,M.J., Budd,J.M., Elliott,L., Eyers,P., Hallam,T.J., Handa,B.K., Hill,C.H., James,M., Lahm,H.W., Lawton,G., Merritt,J.E., Nixon,J.S., Rothlisberger,U., Whittle,A., & Johnson,W.H. (1997) Inhibition of bovine nasal cartilage degradation by selective matrix metalloproteinase inhibitors. Biochem. J., 323, 483-488.
[80] Caputo,C.B., Sygowski,L.A., Wolanin,D.J., Patton,S.P., Caccese,R.G., Shaw,A., Roberts,R.A., & DiPasquale,G. (1987) Effect of synthetic metalloprotease inhibitors on cartilage autolysis in vitro. J. Pharmacol. Exp. Ther., 240, 460-465.
[81] Cawston,T., Plumpton,T., Curry,V., Ellis,A., & Powell,L. (1994) Role of TIMP and MMP inhibition in preventing connective tissue breakdown. Ann. N. Y. Acad. Sci., 732, 75-83.
[82] Seed,M.P., Thomson,T.A., & Gardner,C.R. (1991) Investigation of the role of metalloproteinases in recombinant human interleukin-1 beta-induced degradation of rat femoral head cartilage. Drugs Exp. Clin. Res., 17, 355-361.
[83] Greenwald,R.A. (1999) Thirty-six years in the clinic without an MMP inhibitor. What hath collagenase wrought? Ann. N. Y. Acad. Sci., 878, 413-419.
[84] Elliott,S. & Cawston,T. (2001) The clinical potential of matrix metalloproteinase inhibitors in the rheumatic disorders. Drugs & Aging, 18, 87-99.
[86a] Dube,B., Luke,H.J., Aumailley,M., & Prehm,P. (2001) Hyaluronan reduces migration and proliferation in CHO cells. Biochim. Biophys. Acta, 1538, 283-289.
[89a] Lüke,H.J. & Prehm,P. (1999) Synthesis and shedding of hyaluronan from plasma membranes of human fibroblasts and metastatic and non-metastatic melanoma cells. Biochem. J., 343, 71-75.
[92a] Stein,W.D. (1997) Kinetics of the multidrug transporter (P-glycoprotein) and its reversal. Physiol Rev., 77, 545-590.
[93a] Twentyman,P.R., Rhodes,T., & Rayner,S. (1994) A comparison of rhodamine 123 accumulation and efflux in cells with P- glycoprotein-mediated and MRP-associated multidrug resistance phenotypes. Eur. J. Cancer, 30A, 1360-1369.
[117a] Asplund,T., Versnel,M.A., Laurent,T.C., & Heldin,P. (1993) Human mesothelioma cells produce factors that stimulate the production of hyaluronan by mesothelial cells and fibroblasts. Cancer Res., 53, 388-392.
[118a] Jacobson,A., Rahmanian,M., Rubin,K., & Heldin,P. (2002) Expression of hyaluronan synthase 2 or hyaluronidase 1 differentially affect the growth rate of transplantable colon carcinoma cell tumors. lnt. J. Cancer, 102, 212-219.
[119a] Tufveson,G., Hallgren,R., Johnsson,C., & Wahlberg,J. Use of hyaluronidase in treatment of interstitial edema from organ grafts. Tufveson, Gunnar, Hallgren, Roger, Johnsson, Cecilia, and Wahlberg, Jan. [WO 9808538],
[120a] Engstrom Laurent,A., Feltelius,N., Hallgren,R., & Wasteson,A. (1985) Raised serum hyaluronate levels in scleroderma: an effectof growth factor induced activation of connective tissuecells? Ann. Rheum. Dis., 44, 614-620.
[121a] Lundin,A., Engstrom Laurent,A., Hallgren,R, & Michaelsson,G. (1985) Circulating hyaluronate in psoriasis. Br. J. Dermatol., 112, 663-671.
[122a] Hallgren,R., Eklund,A., Engstrom Laurent,A., & Schmekel,B. (1985) Hyaluronate in bronchoalveolar lavage fluid: a new markerin sarcoidosis reflecting pulmonary disease. Br. Med. J. Clin. Res. Ed., 290, 1778-1781.
[123a] Eklund,A., Hallgren,R., Blaschke,E., Engstrom Laurent,A.P.-U., & Svane,B. (1987) Hyaluronate in bronchoalveolar lavage fluid in sarcoidosisand its relationship to alveolar cell populations. Eur. J. Respir. Dis., 71, 30-36.
[124a] Nettelbladt,O. & Hallgren,R. (1989) Hyaluronan (hyaluronic acid) in bronchoalveolar lavage fluidduring the development of bleomycin-induced alveolitis in therat. Am. Rev. Respir. Dis., 140, 1028-1032.
[125a] Nettelbladt,O., Lundberg,K., Tengblad,A., & Hallgren,R. (1990) Accumulation of hyaluronan in bronchoalveolar lavage fluidis independent of iron-, complement- and granulocyte-depletionin bleomycin-induced alveolitis in the rat. Eur. Respir. J., 3, 765-771.
[126a] Hallgren,R., Gerdin,B., Tengblad,A., & Tufveson,G. (1990) Accumulation of hyaluronan (hyaluronic acid) in myocardial interstitial tissue parallels development of transplantation edema in heart allografts in rats. J. Clin. Invest, 85, 668-673.
[127a] Nettelbladt,O., Scheynius,A., Bergh,J., Tengblad,A., & Hallgren,R. (1991) Alveolar accumulation of hyaluronan and alveolar cellularresponse in bleomycin-induced alveolitis. Eur. Respir. J. , 4, 407-414.
[128a] Bjermer,L., Hallgren,R., Nilsson,K., Franzen,L., Sandstrom,T.S.-B., & Henriksson,R. (1992) Radiation-induced increase in hyaluronan and fibronectin inbronchoalveolar lavage fluid from breast cancer patients issuppressed by smoking. Eur. Respir. J., 5, 785-790.
[129a] Ahrenstedt,O., Knutson,L., Hallgren,R., & Gerdin,B. (1992) Increased luminal release of hyaluronan in uninvolvedjejunum in active Crohn's disease but not in inactive disease orin relatives. Digestion, 52, 6-12.
[130a] Johnsson,C., Hallgren,R., & Tufveson,G. (1993) Recovery of hyaluronan during perfusion of small boweltransplantation reflects rejection. Transplantation, 55, 477-479.
[131a] Waldenstrom,A., Fohlman,J., llback,N.G., Ronquist,G., & Hallgren,R.G.-B. (1993) Coxsackie B3 myocarditis induces a decrease in energy chargeand accumulation of hyaluronan in the mouse heart. Eur. J. Clin. Invest., 23, 277-282.
[132a] Wells,A., Larsson,E., Hanas,E., Laurent,T., & Hallgren,R.T.-G. (1993) Increased hyaluronan in acutely rejecting human kidney grafts. Transplantation, 55, 1346-1349.
[133a] Tufveson,G., Hallgren,R., Johnsson,C., & Wahlberg,J. Use ofhyaluronidase in treatment of interstitial edema from organ grafts. Tufveson, Gunnar, Hallgren, Roger, Johnsson, Cecilia, and Wahlberg, Jan. WO 97-SE1313[WO 9808538 A1]. 1998. PCT lnt. Appl., 18 pp. CODEN: PIXXD2. 24-7-1997. Ref Type: Patent
[134a] Johnsson,C., Hallgren,R., Elvin,A., Gerdin,B., & Tufveson,G. (1999) Hyaluronidase ameliorates rejection-induced edema. TRANSPLANT INTERNATIONAL, 12, 235-243.
[135a] Johnsson,C., Hallgren,R., & Tufveson,G. (2000) Role of hyaluronan in acute pancreatitis. Surgery, 127, 650-658.
[157] Cho, Y. C., You, S. K., Kim, H. J., Cho, C. W., Lee, I. S., and Kang, B. Y. (2010) Xanthohumol inhibits IL-12 production and reduces chronic allergic contact dermatitis. lnt. lmmunopharmacol.
[158] Dorn, C., Weiss, T. S., Heilmann, J., and Hellerbrand, C. (2010) Xanthohumol, a prenylated chalcone derived from hops, inhibits proliferation, migration and interleukin-8 expression of hepatocellular carcinoma cells. lnt. J. Oncol. 36, 435-441.
[159] Dorn, C., Kraus, B., Motyl, M., Weiss, T. S., Gehrig, M., Scholmerich, J., Heilmann, J., and Hellerbrand, C. (2010) Xanthohumol, a chalcon derived from hops, inhibits hepatic inflammation and fibrosis. Mol. Nutr. Food Res.
[160] Lust, S., Vanhoecke, B., VAN, G. M., Boelens, J., VAN, M. H., Kaileh, M., Vanden, B. W., Haegeman, G., Philippe, J., Bracke, M., and Offner, F. (2009) Xanthohumol activates the proapoptotic arm of the unfolded protein response in chronic lymphocytic leukemia. Anticancer Res. 29, 3797-3805.
[161] Cho, Y. C., Kim, H. J., Kim, Y. J., Lee, K. Y., Choi, H. J., Lee, I. S., and Kang, B. Y. (2008) Differential anti-inflammatory pathway by xanthohumol in IFN-gamma and LPS-activated macrophages. Int. Immunopharmacol. 8, 567-573.
[162] Kac, J., Plazar, J., Mlinaric, A., Zegura, B., Lah, T. T., and Filipic, M. (2008) Antimutagenicity of hops (Humulus lupulus L.): bioassay-directed fractionation and isolation of xanthohumol. Phytomedicine. 15, 216-220.
[163] Vanhoecke, B., Derycke, L., Van, M., V, Depypere, H., De, K. D., and Bracke, M. (2005) Antiinvasive effect of xanthohumol, a prenylated chalcone present in hops (Humulus lupulus L.) and beer. Int. J. Cancer 117, 889-895.
[164] Plazar, J., Filipic, M., and Groothuis, G. M. (2008) Antigenotoxic effect of Xanthohumol in rat liver slices. Toxicol. In Vitro 22, 318-327.
[165] Lupinacci, E., Meijerink, J., Vincken, J. P., Gabriele, B., Gruppen, H., and Witkamp,11 R. F. (2009) Xanthohumol from Hop ( Humulus lupulus L.) Is an Efficient Inhibitor of Monocyte Chemoattractant Protein-1 and Tumor Necrosis Factor-alpha Release in LPS-Stimulated RAW 264.7 Mouse Macrophages and U937 Human Monocytes. J. Agric. Food Chem.
[166] Harikumar, K. B., Kunnumakkara, A. B., Ahn, K. S., Anand, P., Krishnan, S., Guha, S., and Aggarwal, B. B. (2009) Modification of the cysteine residues in IkappaBalpha kinase and NF-kappaB (p65) by xanthohumol leads to suppression of NF-kappaB-regulated gene products and potentiation of apoptosis in leukemia cells. Blood 113, 2003-2013.
[167] Lee, S. H., Kim, H. J., Lee, J. S., Lee, I. S., and Kang, B. Y. (2007) Inhibition of topoisomerase I activity and efflux drug transporters' expression by xanthohumol from hops. Arch. Pharm. Res. 30, 1435-1439.
[168] Guerreiro, S., Monteiro, R., Martins, M. J., Calhau, C., Azevedo, I., and Soares, R. (2007) Distinct modulation of alkaline phosphatase isoenzymes by 17beta-estradiol and xanthohumol in breast cancer MCF-7 cells. Clin. Biochem. 40, 268-273.
[169] Stevens, J. F. & Page, J. E. Phytochemistry 2004, 65(10), 1317-1330
[170] Gerhaeuser, C; Frank, N. 2005, 72 pp. Publisher: (Wiley- VCHVerlag GmbH & Co. KGaA, Weinheim, Germany).
[171] Gerhauser, C.; Alt, A.; Heiss, E.; Gamal-Eldeen, A.; Klimo, K.;Knauft, J.; Neumann, I.; Scherf, H-R.; Frank, N.; Bartsch, H. & Becker, H., Molecular Cancer Therapeutics 2002, 1(11), 959-96
[172] Milligan et al., J. Clin. Endocrinol. Metab. 84, 2249-2252
[173] De Keukeleire et al. (2003) J. of Agric. and Food Chem. 51, 4436-4441
[174] Nikolic D., Li Y., Chadwick L-R., Pauli G-F. & van Breemen, R-B, 2005, Mass Spectrom. Mar; 40(3): 289-99, Metabolism of xanthohumol and isoxanthohumol, prenylated flavonoids from hops (Humulus lupulus L.), by human liver microsomes.
[175] Lee H, Wang HW, Su HY & Hao NJ., 1994, Mutagenesis; 9:101-106,The structure-activity relationships of flavonoids as inhibitors of cytochrome P- 450 enzymes in rat liver microsomes and the mutagenicity of 2-amino-3- methyl-imidazo[4,5-f]quinoline.
[176] Stevens JF., Ivancic M., Hsu VL. & Deinzer ML., 1997, Phytochemisty, 44: 1575-1585, Prenylflavonoids from Humulus lupus.
[177] Haas LF., 1995, J Neurol Neurosurg Psychiatry; 58:152, Neurological stamp. Humulus lupulus (hop).

## Claims

1. A compound of the formulas (I): 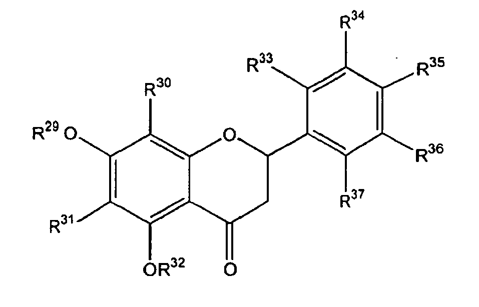 wherein
R29 is H or CH₃
R30 is H or an alkyl-group, such as CH₃, CH₂-CH=CH(CH₃)₂
R31 is H
R32 is H or CH₃O
R33 is H, OH or CH₃O
R34 is H, OH or CH₃O
R35 is H, OH or CH₃O
R36 is H, OH or CH₃O
R37 is H, OH or CH₃O
or a pharmaceutically acceptable salt thereof,
and/or (II) 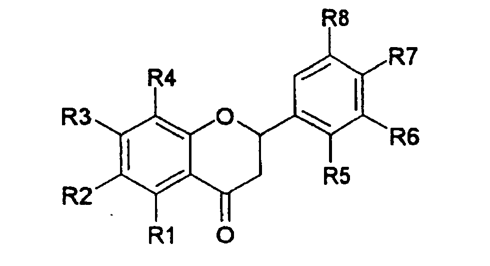 wherein
R1 is H or CH₃O
R2 is H
R3 is H or CH₃
R4 is H or an alkyl-group, such as CH₃, CH₂-CH=CH(CH₃)₂
R5 is H, OH or CH₃O
R6 is H, OH or CH₃O
R7 is H, OH or CH₃O
R8 is H, OH or CH₃O
or a pharmaceutically acceptable salt thereof.
and/or (III) 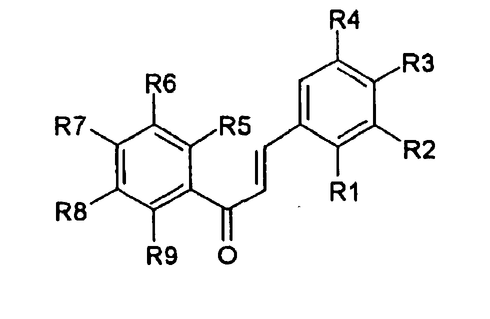 wherein
R1 is H, OH or CH₃O
R2 is H, OH or CH₃O
R3 is H, OH or CH₃O
R4 is H, OH or CH₃O
R5 is H, OH or CH₃O
R6 is H or an alkyl-group, such as CH₃, CH₂-CH=CH(CH₃)₂
R7 is H or CH₃
R8 is H, OH or CH₃O
R9 is H, OH or CH₃O
or a pharmaceutically acceptable salt thereof,
for use in the treatment, prevention (prophylaxis) or healing of a disease which is
(a) associated with or **characterized by** an excess transport of hyaluronan across a lipid bilayer; and/or
(b) associated with or **characterized by** degeneration and/or a destruction of cartilage, preferably in the joint (more preferably the synovial joint); and/or
(c) associated with or **characterized by** proteoglycan (preferably aggrecan) loss, preferably in the joint (more preferably the synovial joint); and/or
(d) associated with or **characterized by** proteolytic cleavage of collagen type II and aggrecan core protein, preferably in the joint (more preferably the synovial joint).

2. The compound of claim 1, which is selected from the following three formulas

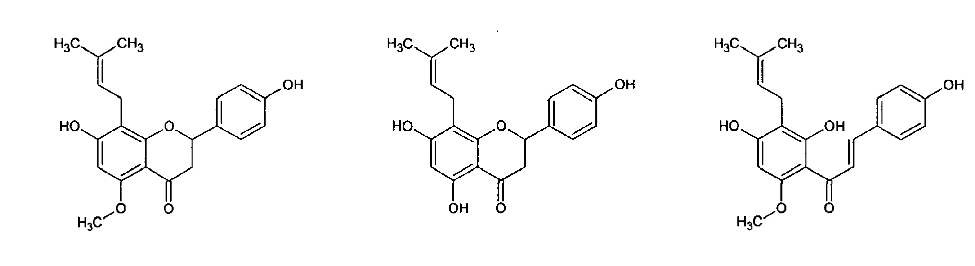

3. A compound as defined in any one of claims 1 or 2, which is comprised in foodstuff.

4. The compound of claim 3, wherein said foodstuff is a beverage.

5. Any of the preceding claims, wherein said disease is osteoarthritis.
